# EUROPÄISCHE PATENTANMELDUNG

(11) **EP 0 794 173 A2**
(43) Veröffentlichungstag der Anmeldung: **10.09.1997**
(21) Anmeldenummer: 97200426.1
(22) Anmeldetag: 14.02.1997
(51) Int. Cl.: C07C 309/17, C11D 1/12, A61K 7/50, A61K 7/075

(54) **Polyglycerinestersulfosuccinate, Verfahren zu ihrer Herstellung und ihre Verwendung**

(30) Priorität: 05.03.1996 DE 19608367
(71) Anmelder: Witco Surfactants GmbH, D-36396 Steinau an der Strasse (DE)
(72) Erfinder: Hintz, Mathias, 36381 Schlüchtern (DE); Irrgang, Bernhard, Dr., 1713 ST Antoni (CH); Köhle, Hans-Jürgen, Dr., 36381 Schlüchtern (DE); Wehner, Winfried, Dr., 36119 Neuhof (DE)

(57) **Zusammenfassung**

Die Erfindung betrifft Fettsäureester-sulfosuccinate, welche hergestellt werden durch Veresterung von Polyglycerinen in erster Stufe mit Fettsäuren FS-COOH, anschließender Addition von Maleinsäureanhydrid in zweiter Stufe, und Sulfierung und gegebenenfalls teilweiser oder völliger Neutralisation des Reaktionsproduktes mit ein oder mehreren organischen und/oder anorganischen Basen.

## Beschreibung

Gegenstand der Erfindung sind neue Polyglycerinfettsäureestersulfosuccinate, Verfahren zu ihrer Herstellung und ihre Verwendung als Reinigungsmittel und für kosmetische Präparate.

Kosmetische Präparate, insbesondere solche, die in den Bereich der Haar- und Körperreinigung fallen, wie Duschbäder, Schaumbäder, Haarshampoos, flüssige Seifen, enthalten als Reinigungskomponenten hauptsächlich Aniontenside wie Carboxylate, Alkylsulfate und Alkylethersulfate, Sulfosuccinate.

Diese Zubereitungen sollen die Hautoberfläche reinigen, vorzugsweise nur den auf ihr anhaftenden Film, welcher aus Körperausscheidungen wie Schweiß und Fetten, Hautschuppen oder abgelagertem Schmutz aus der Umgebung bestehen kann. Die Reinigungsmittel sollen keinesfalls die Haut auslaugen, sie reizen oder ihre natürliche Funktion beeinträchtigen.

Da die Präparate aber bei ihrer häufigen - in den letzten Jahren fast täglichen - Anwendung zu Irritationen der Haut führen können, werden zur Verbesserung der Haut- und Augenschleimhautverträglichkeit häufig sogenannte milde Tenside mitverwendet, wie beispielsweise Betaine, Proteinderivate, Ampholyte, Alkylethercarboxylate und Sulfosuccinate.

Insbesondere für Babypflegemittel und Babyshampoos wird besonderer Wert auf extrem niedrige Gehalte an die Haut und Augenschleimhaut reizende Substanzen gelegt. Die konventionell wegen ihrer ausgezeichneten Reinigigungs- und Schaumbildungseigenschaften verwendeten anionischen Tenside können durch die bekannten milden Co-Tenside in ihrer Irritationswirkung zwar weitgehend gemildert werden, in der Praxis werden aber noch Verbesserungen insbesondere in Richtung auf Augenschleimhautverträglichkeit gefordert.

In hohen Konzentrationen oder allein sind die milden Tenside zwar weitgehend irritationsfrei, zeigen dann jedoch keine praxisgerechten Schäumungs- und Reinigungseigenschaften und unbefriedigende Viskositäten.

Aufgrund des gewachsenen Umweltbewußtseins werden weiterhin Produkte verlangt, welche auf natürlichen nachwachsenden Rohstoffen basieren, keinerlei Toxizität aufweisen und in den kommunalen Kläranlagen schnell und vollständig ohne toxische Zwischenprodukte abgebaut werden können.

Die in der Praxis eingeführten Produkte wie beispielsweise die Mono- und/oder Difettsäureester oder deren Alkoxylate auf Basis von Glycerin bzw. Polyglycerinen weisen zwar niedrige Toxizitäten auf und sind milde die Haut nicht reizende Produkte, welche hinsichtlich ihrer Reinigungswirkung oder Löslichkeit aber nicht den Erfordernissen der Praxis entsprechen.

Aufgabe der vorliegenden Erfindung ist es, diese Nachteile des Standes der Technik zu überwinden und milde, hautfreundliche Reinigungsmittel für Haushalt und Industrie und kosmetische Präparate, insbesondere solche, die in den Bereich der Haar- und Körperreinigung fallen, zur Verfügung zu stellen.

Diese Aufgabe wird gelöst durch die erfindungsgemäße Mitverwendung von Fettsäureester-sulfosuccinaten auf Basis von Polyglycerinen.

Gegenstand der Erfindung sind Fettsäureester-sulfosuccinate der allgemeinen Formel 1 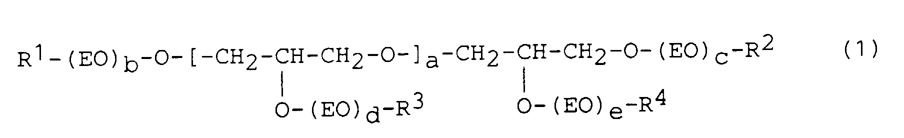 mit der Bedeutung
- R¹ =: FS-CO, worin FS ein gegebenenfalls Mehrfachbindungen enthaltender und/oder gegebenenfalls durch ein oder mehrere OH-Gruppen substituierter Kohlenwasserstoffrest mit 7 - 21 C-Atomen
- R² =: R¹ oder -CO-CH(SO₃⁻X⁺)-CH₂-COO⁻X⁺ oder -CO-CH₂-CH(SO₃⁻X⁺)-COO⁻X⁺
- R³, R⁴ =: gleich oder verschieden = H, R¹, R²,
- EO =: der Rest -CH₂-CH₂-O- mit a, b, c, d, e = 0 - 5
- X⁺ =: H⁺ oder ein Kation ist, welche hergestellt werden durch Veresterung von Polyglycerinen in erster Stufe mit Fettsäuren FS-COOH, anschließender Addition von Maleinsäureanhydrid in zweiter Stufe und Sulfierung und gegebenenfalls teilweiser oder völliger Neutralisation des Reaktionsproduktes mit ein oder mehreren organischen und/oder anorganischen Basen.

Ein weiterer Gegenstand der Erfindung ist ein Verfahren zur Herstellung von Verbindungen der allgemeinen Formel (1).

Weitere Gegenstände der Erfindung sind wäßrige Haarreinigungs- und Pflegemittel, Spülmittel, Allzweckreiniger, Neutralreiniger, Hautpflege- und reinigungsmittel, welche dadurch gekennzeichnet sind, daß sie 1 - 40, vorzugsweise 2 - 30 Gew.-Teile mindestens eine der Verbindungen der allgemeinen Formel (1), definierte Mengen mindestens eines Tensids aus der Gruppe der nichtionischen, amphoteren, zwitterionischen und ionischen Tenside, vorzugsweise 0,1 bis 40 Gew.-Teile, gegebenenfalls 0,1 bis 15 Gew.-Teile einer der üblichen Mittel aus der Gruppe der Zusatz- und Hilfsstoffe, Verdickungsmittel, Duftstoffe, Konservierungsmittel, Farbstoffe, Pflanzenextrakte und gegebenenfalls ad 100 Wasser enthalten.

Weitere Gegenstände der Erfindung sind durch die Ansprüche gekennzeichnet.

Ein Ausgangsstoff für die Herstellung der erfindungsgemässen Verbindungen der allgemeinen Formel (1) ist Polyglycerin der Formel HO-[CH₂-CH(OH)-CH₂-O-]ₐ-CH₂-CH(OH)-CH₂-OH. Die erfindungsgemäß mitverwendeten Polyglycerine sind handelsübliche technische Produkte, welche herstellbar sind nach an sich bekannten Verfahren wie beispielsweise durch basenkatalysierte Polykondensation von Glycerin (Ullmanns Encyklopädie der technischen Chemie, Vol. A 12, 4. Aufl., Verlag Chemie, Weinheim, 1989, S. 477 ff; US-PS 2,605,293 (1952), Shell Development Co.; US-PS 2,810,768 (1957) Shell Development Co.; DE 36 00 388 (Solvay), 1986; DE 34 10 520 (Solvay), 1984). Je nach Herstellungsverfahren weisen diese technischen Produkte unterschiedliche Oligomerisierungsgrade und Verteilungen auf.

Diese Polyglycerine können in einem ersten Schritt mit einem bzw. zwei Mol Fettsäure bzw. deren Ester nach den bekannten Methoden zum Ester umgesetzt werden (G. Jakobson, Fette, Seifen, Anstrichmittel, **88**, (1986), S. 101 - 105). Erfindungsgemäß bevorzugt sind die Monoester.

Als Säuren werden die monofunktionellen Fettsäuren bzw. Fettsäuregemische mit 8 - 22 C-Atomen - bevorzugt die natürlich vorkommenden - verwendet wie z. B. Kokosfettsäuren, Palmkernfettsäuren oder Palmfettsäuren, Talgfettsäuren.

Erfindungsgemäß bevorzugt werden die einbasischen Säuren mit 8 - 18 C-Atomen wie Ölsäure, Linolsäure, Linolensäure, Ricinolsäure, Kokosfettsäure oder deren Mischungen verwendet.

Diese Ester werden in einem zweiten Schritt nach an sich bekannten Verfahren (J. Am. Oil Chemist's Soc., 1956, 33, 571, EP-A-0 334 482) mit Alkylenoxiden wie insbesondere Ethylenoxid zu den Polyglykoletherestern umgesetzt, wobei im statistischen Mittel pro Hydroxylgruppe 0 - 5 Mol Alkylenoxid angelagert werden.

Die Polyglycerine können in erster Stufe auch noch nach den oben angeführten bekannten Verfahren alkoxyliert und in zweiter Stufe mit 1 oder 2 Mol Fettsäure zum Fettsäureester umgesetzt werden.

Die Reaktion zum Sulfosuccinat erfolgt mit Maleinsäureanhydrid und anschließender Sulfierung mit Natriumsulfit nach an sich bekannten Verfahren (DE-OS 27 00 072).

Dabei wird pro Mol umzusetzende Hydroxylgruppe mit maximal einem Mol Maleinsäureanhydrid bei 60 - 80 °C bis zur vollständigen Reaktion des Anhydrids gerührt. Der Maleinsäurehalbester wird anschließend in eine wäßrige Natriumsulfit-Lösung gegeben (1 Equivalent Sulfit pro Equivalent Halbester) und bei 60 - 80 °C sulfiert, bis das Sulfit vollständig abreagiert ist. Dann wird das wäßrige Produkt pH-neutral eingestellt.

Im allgemeinen wird erfindungsgemäß das Verfahren so geführt, daß in erster Stufe das Polyglycerin mit der oder den Fettsäuren, gegebenenfalls unter Mitverwendung eines Veresterungskatalysators, bei 180 - 210 °C unter Entfernung des Reaktionswassers verestert, dieser gegebenenfalls in zweiter Stufe in Gegenwart eines basischen Katalysators bei 80 - 150 °C und 1 - 10 bar mit Alkylenoxid zum Alkoxylat umgesetzt wird, welches dann durch Addition von Maleinsäureanhydrid bei 60 - 80 °C zum Halbester und in letzter Stufe bei 60 - 80 °C mit wäßriger Natriumsulfitlösung zum Salz der entsprechenden Sulfonsäure umgesetzt wird.

Die erfindungsgemäßen Verbindungen sind durch die allgemeine Formel (1) in idealisierter Form dargestellt. Die technischen Mischungen enthalten neben den angegebenen Monofettsäureestern oder Difettsäureestern noch Anteile der Ausgangsprodukte, und auch die angegebenen Alkoxylierungsgrade sind statistische Mittelwerte.

Für die erfindungsgemäße Verwendung ist eine Reinigung nicht erforderlich, das heißt, die technischen Mischungen sind als solche direkt weiter verwendbar.

Die erfindungsgemäßen Mischungen für Reinigungszwecke und Kosmetik liegen im allgemeinen als wäßrige Mittel oder als wäßrige alkoholische Lösungen, Cremes, Emulsion, Gele vor und können zur Anpassung an den vorgesehenen Anwendungszweck noch die jeweils üblichen Hilfs- und Zusatzstoffe enthalten, welche für die Herstellung von Reinigungsmitteln und kosmetischen Präparaten im Bereich der Haar- und Körperreinigung, das heißt für Duschbäder, Schaumbäder, Shampoos, flüssige Seifen, Babypflege- und waschmittel sowie für milde Reinigungsmittel wie Geschirrspülmittel, Allzweckreiniger oder Neutralreiniger für manuelle Anwendung üblich sind, mitverwendet werden.

Für die kosmetischen Anwendungen können die auf diesen Gebieten üblichen Tenside, Duftstoffe, Konservierungsmittel, Farbstoffe, Pflanzenextrakte bzw. sonstige kosmetische Additive mitverwendet werden.

Als Tenside kommen neben den bekannten Betainen, amphoteren und nichtionischen Verbindungen für die reinigenden Rezepturen insbesondere die anionischen Tenside wie Carboxylate, Alkylsulfate, Alkylethersulfate, Alkylsulfonate, Alkylethersulfonate, Alkylsulfosuccinate in Betracht. Erfindungsgemäß bevorzugt werden Alkylamidobetaine wie REWOTERIC® ¹⁾ AM B 13 und AM B 14, sowie AM B 45, Carboxyglycinate wie REWOTERIC® AM 2 C NM, Carboxypropionate wie REWOTERIC® AM KSF 40, Sulfobetaine wie REWOTERIC® AM CAS, anionische Tenside wie Ethersulfate REWOPOL® ²⁾ NL 2, Ethercarboxylate wie REWOPOL® CLN 100, Sulfosuccinate wie REWOPOL® SB FA 30, REWOPOL® SBZ, REWODERM® ³⁾ SPS, nichtionische Tenside wie Glycerinfettsäureesterethoxylate wie REWODERM® ES 90, Glycerinmonostearat wie REWOMUL® ⁴⁾ MG, Cetylalkohol
(¹⁾, ²⁾, ³⁾, ⁴⁾ = Warenzeichen der Fa. REWO Chemische Werke GmbH, Steinau an der Straße).

Neben den erfindungsgemäßen Verbindungen, welche in Mengen von 1 - 40 Gew.-Teilen, insbesondere 2 - 30 Gew.-Teilen, bei Shampoos und 1 - 7 Gew.-Teilen, vorzugsweise 1 - 5 Gew.-Teilen, bei Cremes sowie 1 - 10, vorzugsweise 1,5 - 8 Gew.-Teilen, bei Reinigungsmitteln eingesetzt werden, werden die anderen Tenside bei den Shampoos üblicherweise in Mengen von 1 - 40 Gew.-Teilen, vorzugsweise 2 - 30 Gew.-Teilen, bei den Cremes in Mengen von 1 - 10 Gew.-Teilen, vorzugsweise 2 - 8 Gew.-Teilen, mitverwendet.

Als Verdickungsmittel werden 0,1 - 10 Gew.-Teile der auf diesem Gebiet üblichen Verbindungen wie Glycerinfettsäureesterethoxylate, Fettalkoholethoxylate, Fettsäurealkylolamide und die üblichen Alkali-, Erdalkali- und Ammoniumsalze, welche bei 20 °C in Mengen von mindestens 1 Gew.-% in Wasser löslich sind, wie insbesondere NaCl, NH₄Cl, eingesetzt. Verdickungsmittel sind Stoffe oder Verbindungen, die eingesetzt werden, um die Viskositäten von Mischungen oder Formulierungen zu erhöhen.

Die in den nachfolgenden Beispielen angeführten Analysenwerte werden nach den folgenden auf diesem Gebiet allgemein üblichen Methoden bestimmt:

### Säurezahl (SZ)

Die Säurezahl ist ein Maß für den Gehalt eines Fettes oder von technischen Fettsäuren an freien Säuren und gibt die Milligramm Kaliumhydroxid an, die notwendig sind, um 1 Gramm Substanz zu neutralisieren.

Die Werte werden bestimmt nach der DGF-Einheitsmethode C-V4.

### Gehalt an Trockensubstanz

Der Gehalt wird bestimmt durch Erhitzen und Trocknen bei 105 °C bis zur Gewichtskonstanz.

### Hydroxylzahl (OHZ)

Die Hydroxylzahl dient zur Ermittlung des Gehalts an Hydroxylgruppen und gibt die Anzahl Milligramm Kaliumhydroxid an, die notwendig ist, um die von 1 Gramm Fett bei der Acetylierung verbrauchte Essigsäure zu neutralisieren (mg KOH/g).

### Benzavlon-WAS

Der in den nachfolgenden Beispielen angegebene Gehalt an waschaktiver Substanz (B-WAS) wird durch die übliche Zweiphasentitration mit Benzalkoniumchlorid (Benzavlon) gegen Methylenblau als Indikator titriert (vgl. S. R. Epton, Nature (London) 160, 1967, S. 795).

### Beispiele

### 1. Synthese von Polyglycerinestern

### Beispiel 1.1

Ester aus Triglycerin (Solvay) und Fettsäure HK 12-18

254.2 g (1.1 mol) Triglycerin, 233.1 g (1.1 mol) Fettsäure HK 12-18 und 1.0 g (0.4 %) hypophosphorige Säure wurden in einem Reaktionsgefäß, ausgerüstet mit Thermometer, Rührer, Kühler und Wasserabscheider, zusammengegeben und unter Stickstoffatmosphäre auf 190 °C erhitzt und das entstehende Reaktionswasser abdestilliert. Bei Erreichen der Temperatur wurde Vakuum (ca. 50 mbar) angelegt und Reste des Reaktionswassers entfernt. Bei Erreichen einer Säurezahl von ca. 5 mg KOH/mg wurde bei 180 °C für eine Stunde nachreagiert und abgekühlt.

Das Reaktionsprodukt wies die folgenden Analysendaten auf:

| | |
|---|---|
| VZ | 135.2 mg KOH/g |
| OHZ | 344.0 mg KOH/g |
| SZ | 2.5 mg KOH/g |

### Beispiel 1.2

### Ester aus Triglycerin (Solvay) und C 12 Fettsäure

331.7 g (1.4 mol) Triglycerin, 277 g (1.4 mol) C 12 Fettsäure und 2.4 g hypophosphorige Säure wurden in einem Reaktionsgefäß, ausgerüstet mit Thermometer, Rührer, Kühler und Wasserabscheider, zusammengegeben und unter Stickstoffatmosphäre auf 205 °C erhitzt und das entstehende Reaktionswasser abdestilliert. Bei Erreichen der Temperatur wurde Vakuum (ca. 50 mbar) angelegt und Reste des Reaktionswassers entfernt. Bei Erreichen einer Säurezahl von ca. 5 mg KOH/mg wurde bei 180 °C für eine Stunde nachreagiert und abgekühlt.

Das Reaktionsprodukt wies die folgenden Analysendaten auf:

| | |
|---|---|
| VZ | 129.6 mg KOH/g |
| OHZ | 516.2 mg KOH/g |
| SZ | 4.5 mg KOH/g |

### Beispiel 1.3

Ester aus C 12 Fettsäure und Polyglycerin T (Handelsprodukt der Fa. Solvay; a in der allgemeinen Formel (1) = 2 - 6)

295 g (0.9 mol) Triglycerin, 188 g (0.9 mol) C 12 Fettsäure HK und 0.9 g hypophosphorige Säure wurden in einem Reaktionsgefäß, ausgerüstet mit Thermometer, Rührer, Kühler und Wasserabscheider, zusammengegeben und unter Stickstoffatmosphäre auf 200 °C erhitzt und das entstehende Reaktionswasser abdestilliert. Bei Erreichen der Temperatur wurde Vakuum (ca. 50 mbar) angelegt und Reste des Reaktionswassers bei 180 C entfernt. Bei Erreichen einer Säurezahl von ca. 5 mg KOH/mg wurde bei 180 °C für eine Stunde nachreagiert und abgekühlt.

Das Reaktionsprodukt wies die folgenden Analysendaten auf:

| | |
|---|---|
| VZ | 119.2 mg KOH/g |
| OHZ | 403.1 mg KOH/g |
| SZ | 1.2 mg KOH/g |

### Beispiel 1.4

Ester aus Polyglycerin T (Solvay) und Fettsäure C 8-18

786 g (2.5 mol) Polyglycerin, 733.6 g (3.75 mol) Fettsäure C 8-18 und 5.0 g hypophosphorige Säure wurden in einem Reaktionsgefäß, ausgerüstet mit Thermometer, Rührer, Kühler und Wasserabscheider, zusammengegeben und unter Stickstoffatmosphäre auf 205 °C erhitzt und das entstehende Reaktionswasser abdestilliert. Bei Erreichen der Temperatur wurde Vakuum (ca. 20 mbar) angelegt und Reste des Reaktionswassers entfernt. Bei Erreichen einer Säurezahl von ca. 5 mg KOH/mg wurde bei 180 °C für eine Stunde nachreagiert und abgekühlt.

Das Reaktionsprodukt wies die folgenden Analysendaten auf:

| | |
|---|---|
| VZ | 149.6 mg KOH/g |
| OHZ | 409.0 mg KOH/g |
| SZ | 4.9 mg KOH/g |

### 2. Ethoxylierung

### Beispiel 2.1

490.3 g (0.73 mol) Polyglycerinester aus Beispiel 1.4 wurden nach Zusatz von 2.8 g KOH (100 %ig) bei ca. 140 °C im Laborautoklaven portionsweise mit insgesamt 310 g (7.0 mol) Ethylenoxid so umgesetzt, daß der Druck maximal 5 bar betrug.

Analysenzahlen:

| | |
|---|---|
| VZ | 136.3 mg KOH/ g |
| OHZ | 234 mg KOH/g |

### Beispiel 2.2

620.8 g (1.2 mol) Polyglycerinester aus Beispiel 1.4 wurden nach Zusatz von 1.9 g KOH (100 %ig) bei ca. 140 °C im Laborautoklaven portionsweise mit insgesamt 533 g (12.1 mol) Ethylenoxid so umgesetzt, daß der Druck maximal 5 bar betrug.

Analysenzahlen:

| | |
|---|---|
| VZ | 95.7 mg KOH/g |
| OHZ | 160.4 mg KOH/g |

### 3. Halbesterbildung und Sulfierung

### Beispiel 3.1

489 g (0.5 mol) des Polyglycerinesterethoxylats aus Beispiel 2.2 wurden bei 70 - 80 °C unter N2 mit 50.2 g (0.5 mol) Maleinsäureanhydrid versetzt und 3 h bei dieser Temperatur reagiert. Das so erhaltenene Produkt wurde langsam zu einer Lösung von 49.7 g (0.25 mol) Natriumbisulfit in 580 g Wasser bei 80 - 85 °C getropft. Die Lösung wies nach Einstellung folgende Analysendaten auf:

| | |
|---|---|
| Trockenrückstand | 49.6 % |
| Benzavlon-WAS | 32.3 % |
| pH-Wert (5%) | 6.3 |

### Beispiel 3.2

489 g (0.5 mol) des Polyglycerinesterethoxylats aus Beispiel 2.2 wurden bei 70 - 80 °C unter N2 mit 87.9 g (0.88 mol) Maleinsäureanhydrid versetzt und 3 h bei dieser Temperatur reagiert. Das so erhaltenene Produkt wurde langsam zu einer Lösung von 87.0 g (0.44 mol) Natriumbisulfit in 1000 g Wasser bei 80 - 85 °C getropft. Die Lösung wies nach Einstellung folgende Analysendaten auf:

| | |
|---|---|
| Trockenrückstand | 50.2 % |
| Benzavlon-WAS | 43.7 % |
| pH-Wert (5%) | 6.2 |

### Beispiel 3.3

389 g (0.75 mol) des Polyglycerinesters aus Beispiel 1.1 wurden bei 70 - 80 °C unter N2 mit 92.5 g (0.94 mol) Maleinsäureanhydrid versetzt und 2 h bei dieser Temperatur reagiert.

Das so erhaltenene Produkt wurde langsam zu einer Lösung von 91.6 g (0.47 mol) Natriumbisulfit in 570 g Wasser bei 80 - 85 °C getropft. Die Lösung wies nach Einstellen folgende Analysendaten auf:

| | |
|---|---|
| Trockenrückstand | 39.2 % |
| Benzavlon-WAS | 28.7 % |
| pH-Wert (5%) | 6.1 |

### Beispiel 3.4

427 g (0.8 mol) des Polyglycerinesters aus Beispiel 1.1 wurden bei 70 - 80 °C unter N2 mit 122 g (1.2 mol) Maleinsäureanhydrid versetzt und 2 h bei dieser Temperatur reagiert.

Das so erhaltenene Produkt wurde langsam zu einer Lösung von 121 g (0.6 mol) Natriumbisulfit in 1000 g Wasser bei 80 - 85 °C getropft. Die Lösung wies nach Einstellen folgende Analysendaten auf:

| | |
|---|---|
| Trockenrückstand | 40.7 % |
| Benzavlon-WAS | 36.0 % |
| pH-Wert (5%) | 5.9 |

### Beispiel 3.5

427 g (0.8 mol) des Polyglycerinesters aus Beispiel 1.4 wurden bei 70 - 80°C unter N2 mit 126 g (1.2 mol) Maleinsäureanhydrid versetzt und 4 h bei dieser Temperatur reagiert.

Das so erhaltenene Produkt wurde langsam zu einer Lösung von 125 g (0.6 mol) Natriumbisulfit in 1000 g Wasser bei 80 - 85 °C getropft. Die Lösung wies nach Einstellen folgende Analysendaten auf:

| | |
|---|---|
| Trockenrückstand | 48.3 % |
| Benzavlon-WAS | 48.0 % |
| pH-Wert (5%) | 5.4 |

### Beispiel 3.6

443 g (1.1 mol) Triglycerinlaurat wurden bei 70 - 80 °C unter N2 mit 182 g (1.9 mol) Maleinsäureanhydrid versetzt und 2 h bei dieser Temperatur reagiert.

Das so erhaltenene Produkt wurde langsam zu einer Lösung von 165 g (0.9 mol) Natriumbisulfit in 1000 g Wasser bei 80 - 85 °C getropft. Die Lösung wies nach Einstellen folgende Analysendaten auf:

| | |
|---|---|
| Trockenrückstand | 50.6 % |
| Benzavlon-WAS | 49.6 % |
| pH-Wert (5%) | 5.7 |

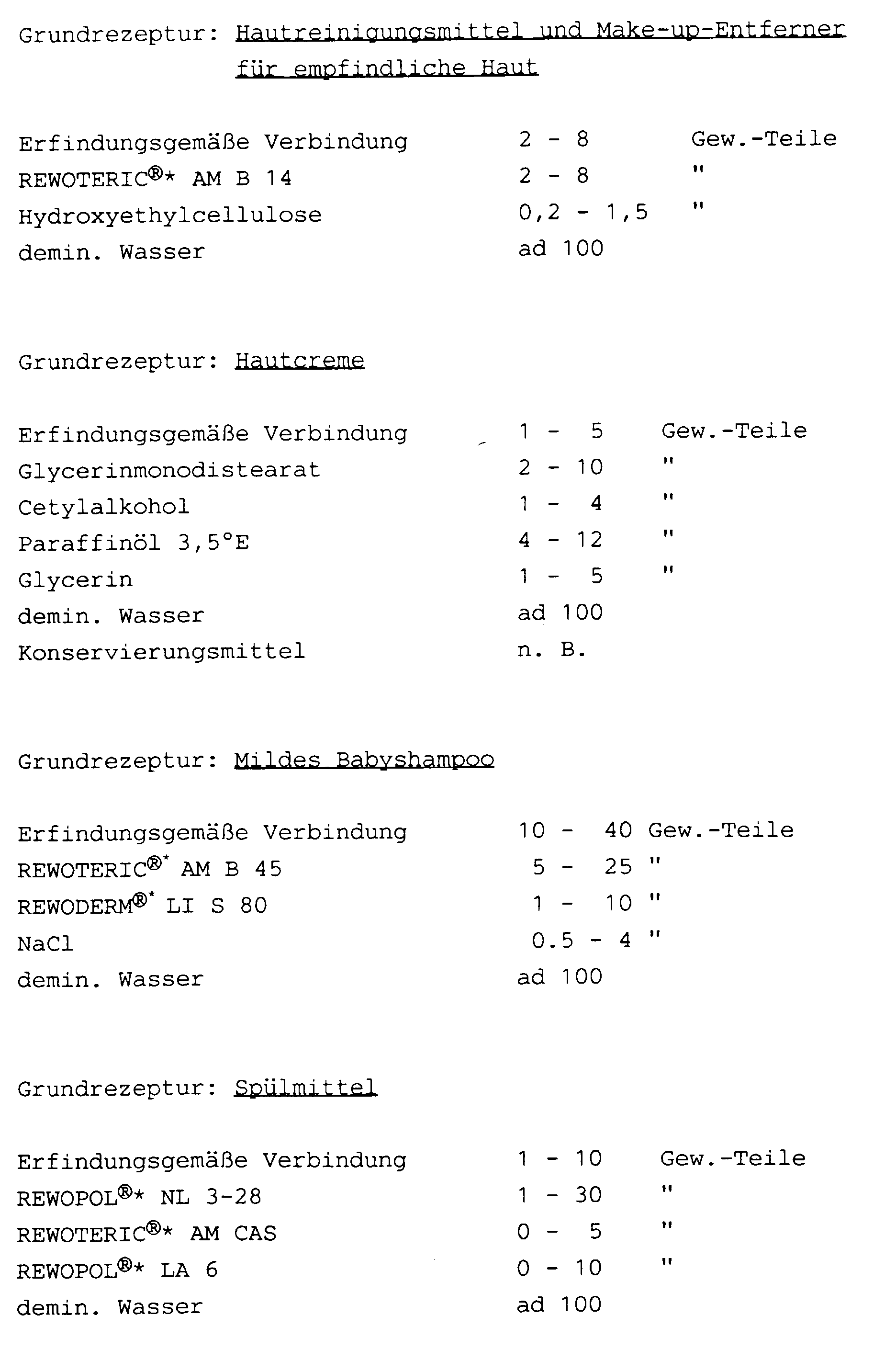

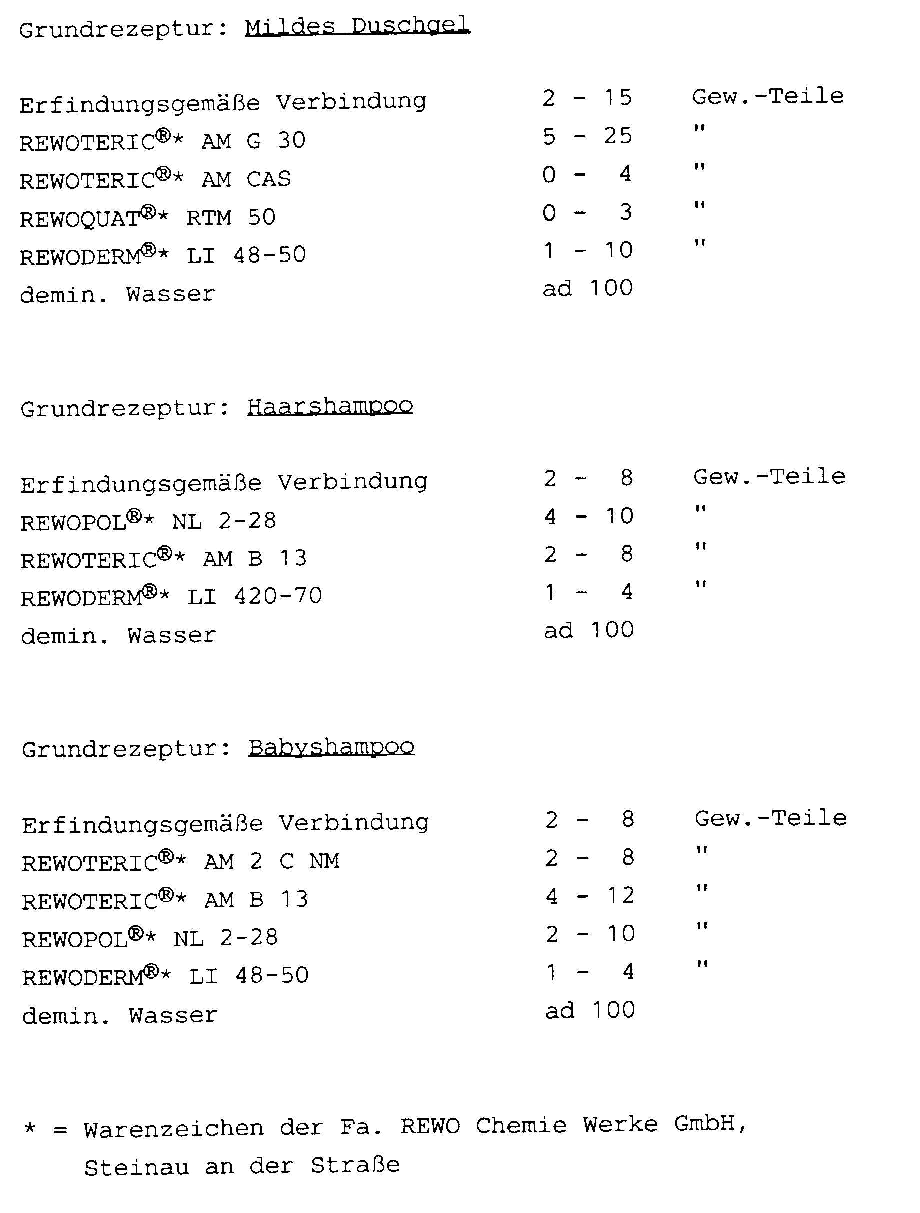

### Kosmetische Formulierungen

Die nachfolgenden Formulierungen können durch einfaches Einrühren der Rezepturbestandteile in Wasser hergestellt werden. Alle Rezepturen werden in Gewichtsprozenten auf Feststoff berechnet angegeben.

### Erklärung der eingesetzten Tenside nach INCI-Namen

| | |
|---|---|
| REWOMUL® MG | Glyceryl Stearate |
| REWOPOL® SB FA 30 | Disodium Laureth Sulfosuccinate |
| REWOTERIC® AM B 45 | Cocamidopropylbetaine |
| REWOTERIC® AM B 13 | Cocamidopropylbetaine |
| REWOTERIC® AM B 14 | Cocamidopropylbetaine |
| REWOTERIC® AM 2 C NM | Disodium Cocoamphodiacetate |
| REWOPOL® SBZ | Disodium PEG-4 Cocoamido MIPA-Sulfosuccinate |
| REWODERM® ES 90 | PEG-7 Glyceryl Cocoate |
| REWODERM® LI S 80 | PEG-200 Hydrogenated Glyceryl Palmitate (and) PEG-7 Glyceryl Cocoate |
| REWOPOL® NL 2-28 | Sodium Laureth Sulfate |
| REWOTERIC® AM G 30 | Disodium Lauroamphodiacetate (and) Sodium Lauryl Sulfate (and) Hexylene Glycol |
| REWODERM® LI 48-50 | PEG-80 Glyceryl Tallowate |
| REWODERM® LI 420 | PEG-200 Glyceryl Tallowate |
| REWODERM® LI 420-70 | PEG-200 Glyceryl Tallowate mod. |
| REWOTERIC® AM CAS | Cocoamidopropyl Hydroxysultaine |
| REWOPAL® LA 6 | Laureth-6 |
| REWOQUAT® RTM 50 | Ricinoleamidopropyltrimonium Methosulfate |

### Analysenmethoden

**Calciumhärteverträglichkeit:** DIN 53 905

**Hautverträglichkeit:** RBC-Test; W.J.W. Pape, U. Pfannenbecker, und U. Hoppe, Mol. Toxicol. **1987**, 1, 525.

| Bewertung: | |
|---|---|
| L/D-Wert | Einstufung |
| > 100 | nicht reizend |
| > 10 | leicht reizend |
| > 1 | mäßig reizend |
| > 0.1 | reizend |
| < 0.1 | stark reizend |

**Oberflächenspannung:** Dr. R. Hensch; Fette, Seifen, Anstrichmittel, **1970**, 72, 969

**Viskosität:** Brookfield Rotationsviskosimeter (Becher und Spindel) gemessen bei 20 °C nach den Angabe des Geräteherstellers

**Schaumvermögen:** nach Ross-Miles (DIN 53902 Teil 2)

**Tabelle 1**

| Verbindung gemäß Beispiel | a | b+c+d+e | x*R¹ | y*(R²+ R³ + R⁴)= R¹ |
|---|---|---|---|---|
| | | | | z*(R²+ R³ + R⁴)= R⁵ |
| | | | | m*(R²+ R³ + R⁴)= H |
| 3.1 | 3-6 | 10 | 1 | y 0.5 |
| | | | | z 1 |
| | | | | m 2.5 |
| 3.2 | 3-6 | 10 | 1 | y 0.5 |
| | | | | z 1.75 |
| | | | | m 1.75 |
| 3.3 | 3 | 0 | 1 | y 0 |
| | | | | z 1.25 |
| | | | | m 2.75 |
| 3.4 | 3 | 0 | 1 | y 0 |
| | | | | z 1.5 |
| | | | | m 2.5 |
| 3.5 | 3-6 | 0 | 1 | y 0.5 |
| | | | | z 1.5 |
| | | | | m 2 |
| 3.6 | 3 | 0 | 1 | y 0 |
| | | | | z 1.75 |
| | | | | m 2.25 |

R⁵ = -CO-CH(SO₃⁻X⁺)-CH₂-COO⁻X⁺ od. -CO-CH₂-CH(SO₃⁻X⁺)-COO⁻X⁺

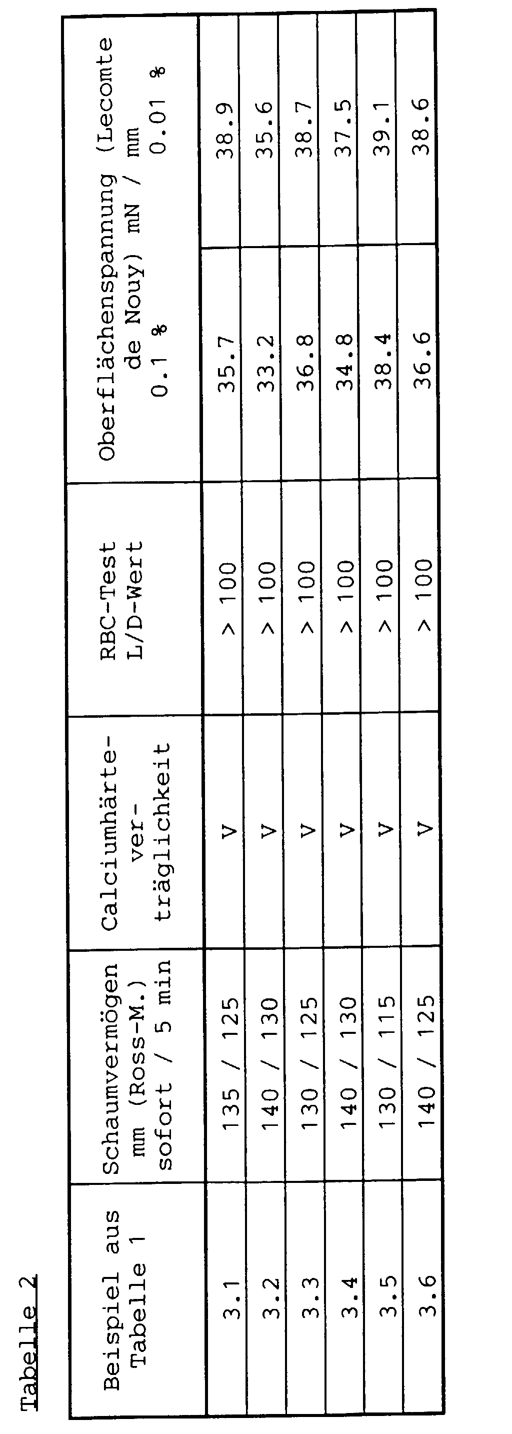

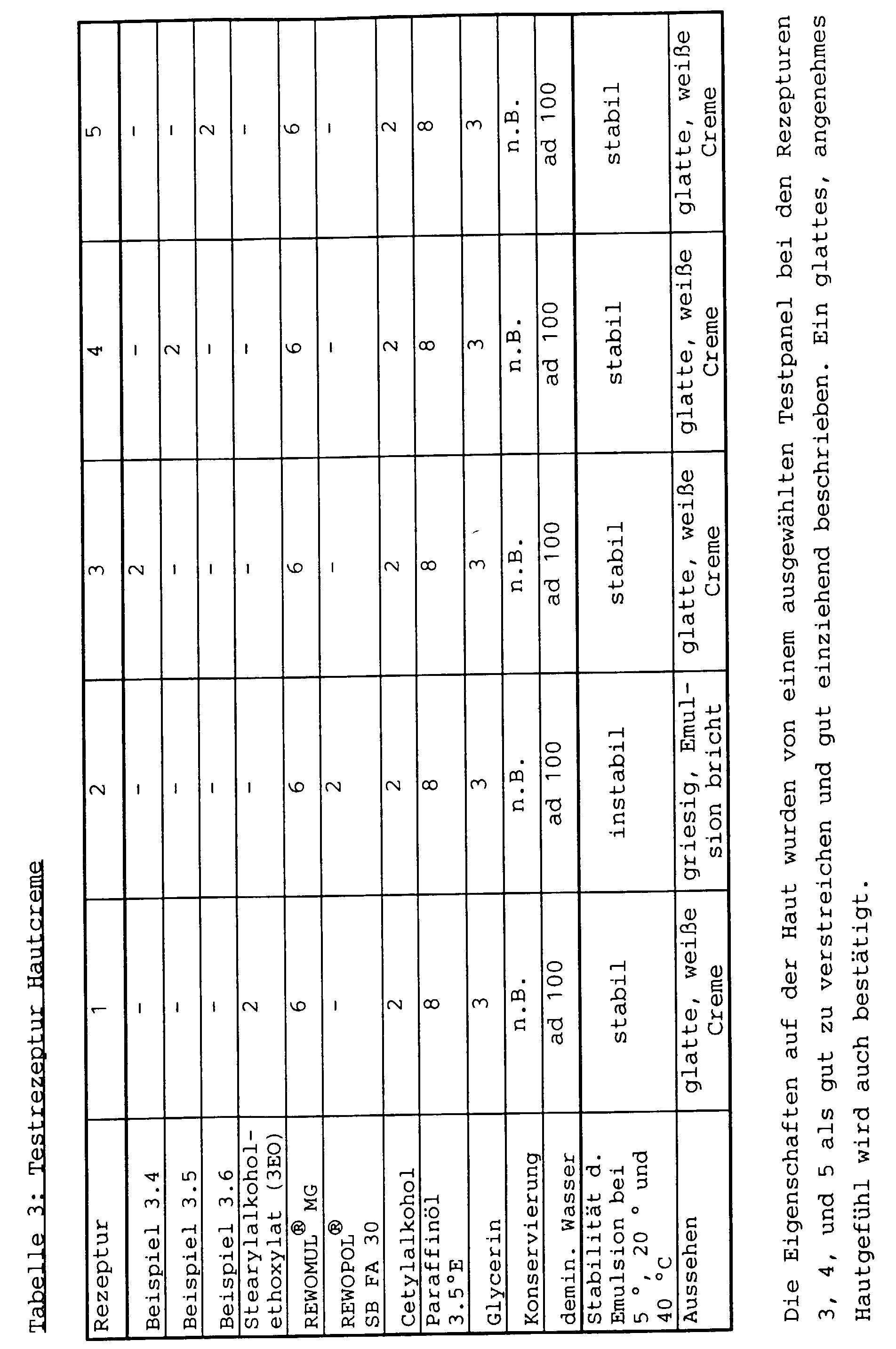

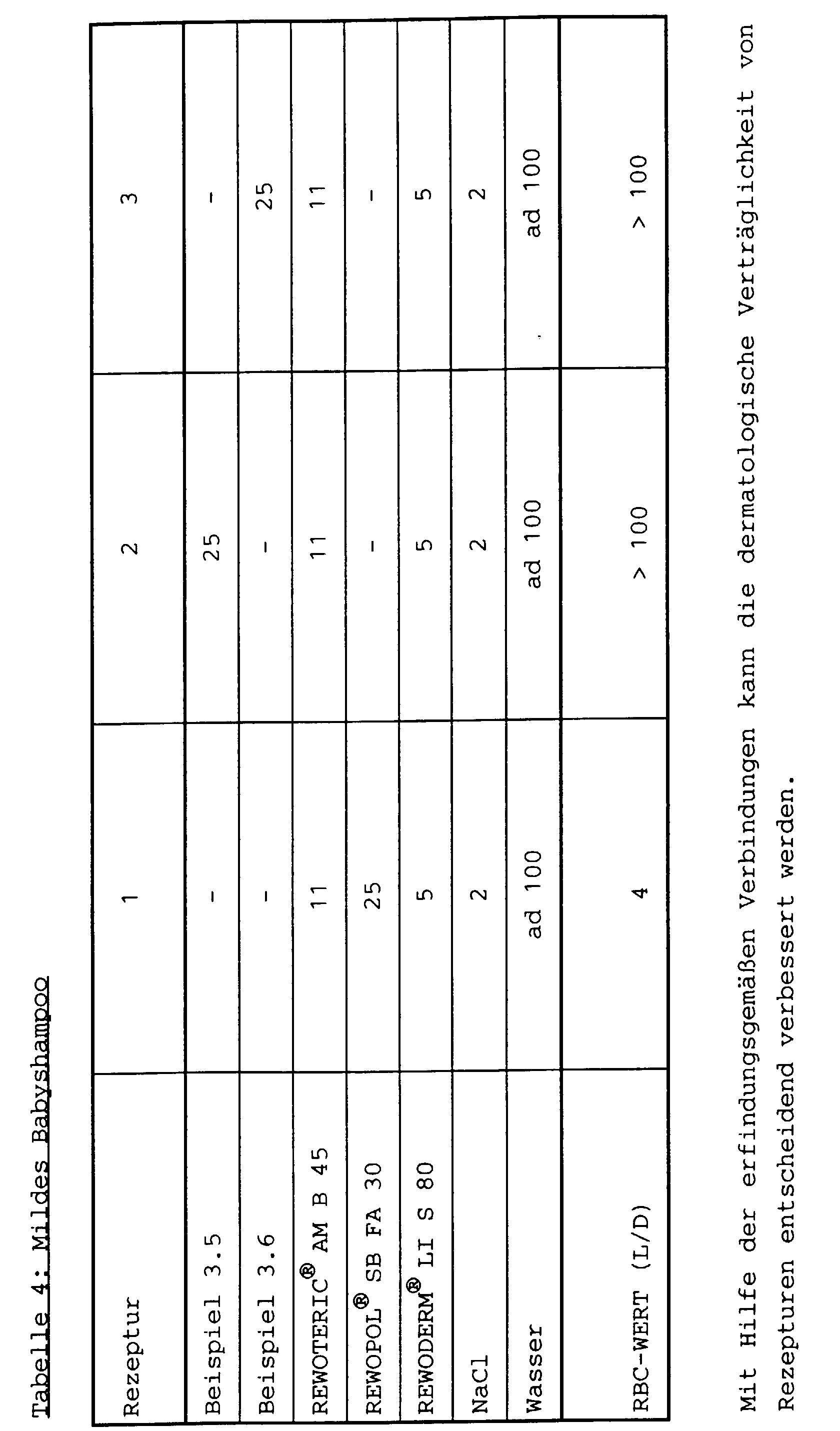

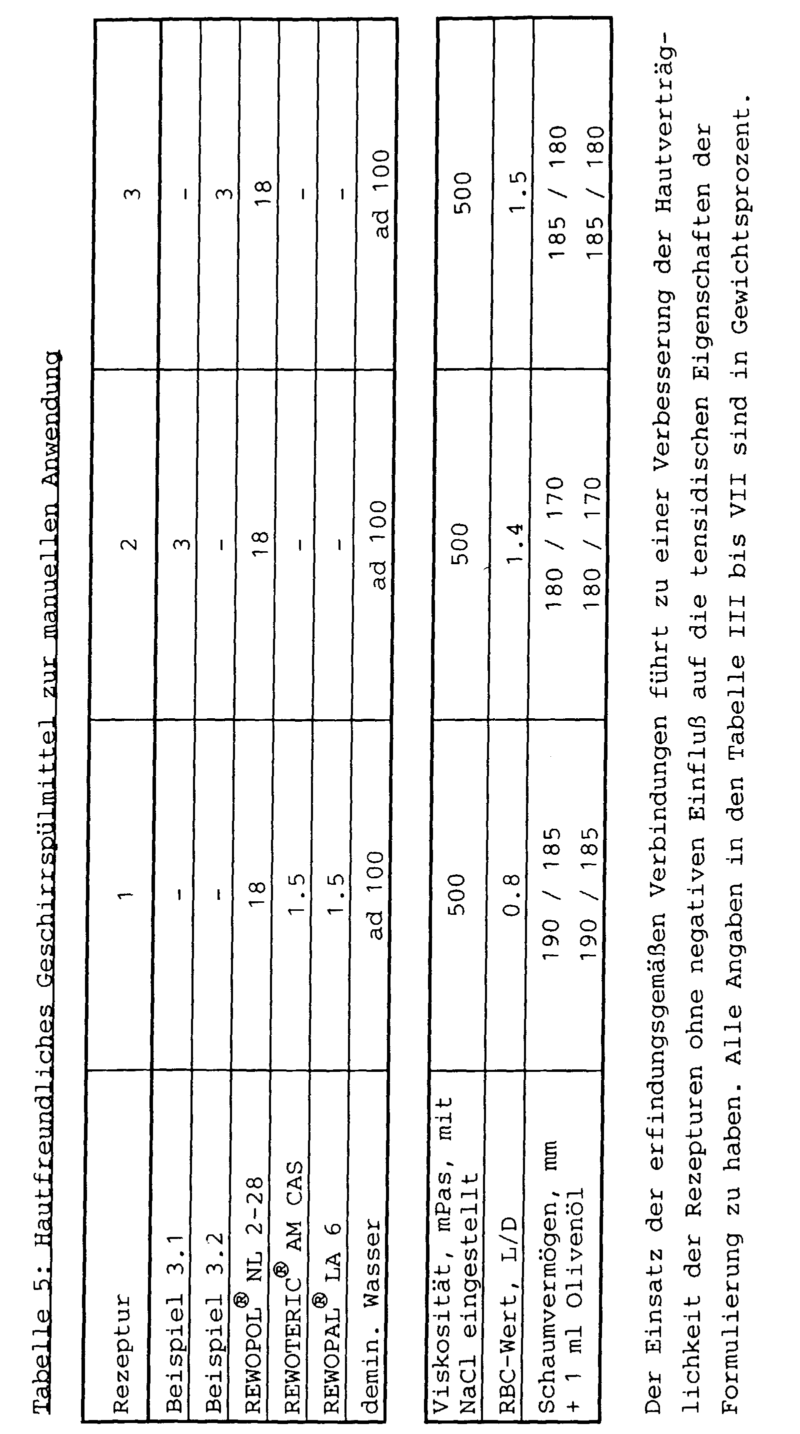

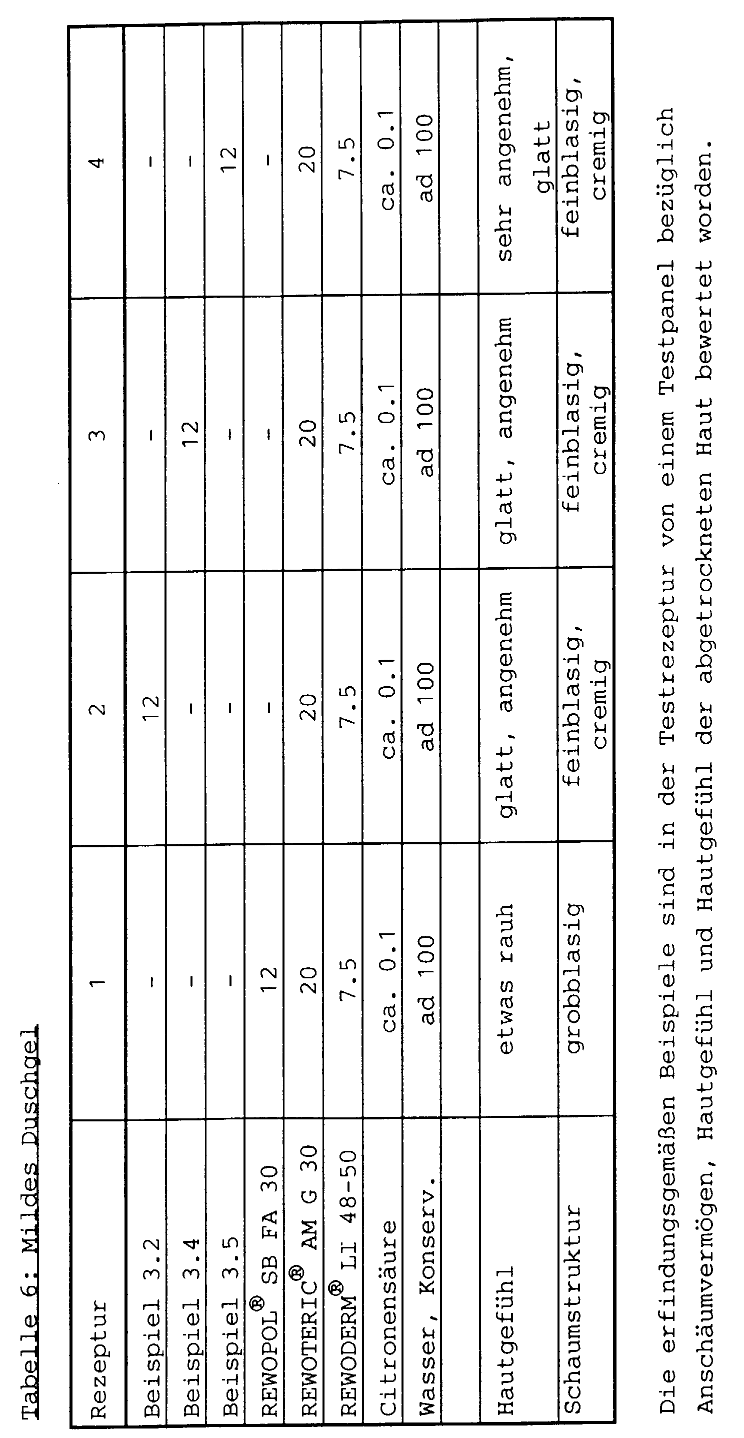

## Patentansprüche

1. Fettsäureester-sulfosuccinate der allgemeinen Formel 1 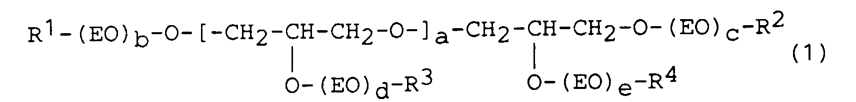 mit der Bedeutung
R¹ = FR-CO, worin FS ein gegebenenfalls Mehrfachbindungen enthaltender und/oder gegebenenfalls durch ein oder mehrere OH-Gruppen substituierter Kohlenwasserstoffrest mit 7 - 21 C-Atomen
R² = R¹ oder -CO-CH(SO₃⁻X⁺)-CH₂-COO⁻X⁺ oder -CO-CH₂-CH(SO₃⁻X⁺)-COO⁻X⁺
R³, R⁴ = gleich oder verschieden = H, R¹, R²,
EO = der Rest -CH₂-CH₂-O- mit a, b, c, d, e = 0 - 5
X⁺ = H⁺ oder ein Kation ist, welche hergestellt werden durch Veresterung von Polyglycerinen in erster Stufe mit Fettsäuren FS-COOH, anschließender Addition von Maleinsäureanhydrid in zweiter Stufe und Sulfierung und gegebenenfalls teilweiser oder völliger Neutralisation des Reaktionsproduktes mit ein oder mehreren organischen und/oder anorganischen Basen.

2. Verbindungen der allgemeinen Formel (1) gemäß Anspruch 1, dadurch gekennzeichnet, daß
R¹ = FS-CO mit FS = Kokosfettsäure und
R², R³, R⁴ = mindestens einmal 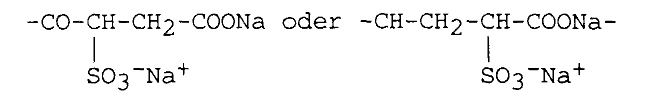 und ansonsten = H bedeuten und
a = 1 - 4 und b, c, d, e = O sind.

3. Verfahren zur Herstellung der Verbindungen der allgemeinen Formel (1) 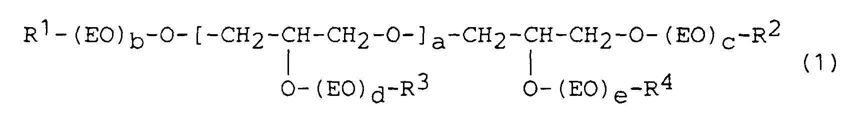 mit der Bedeutung
R¹ = FR-CO, worin FS ein gegebenenfalls Mehrfachbindungen enthaltender und/oder gegebenenfalls durch ein oder mehrere OH-Gruppen substituierter Kohlenwasserstoffrest mit 7 - 21 C-Atomen
R² = R¹ oder -CO-CH(SO₃⁻X⁺)-CH₂-COO⁻X⁺ oder -CO-CH₂-CH(SO₃⁻X⁺)-COO⁻X⁺
R³, R⁴ = gleich oder verschieden = H, R¹, R²,
EO = der Rest -CH₂-CH₂-O- mit a, b, c, d, e = 0 - 5
X⁺ = H⁺ oder ein Kation ist, welche hergestellt werden durch Veresterung von Polyglycerinen in erster Stufe mit Fettsäuren FS-COOH, anschließender Addition von Maleinsäureanhydrid in zweiter Stufe und Sulfierung und gegebenenfalls teilweiser oder völliger Neutralisation des Reaktionsproduktes mit ein oder mehreren organischen und/oder anorganischen Basen.

4. Wäßriges Dusch- und Haarshampoo, enthaltend
a) 1- 40 Gew.-Teile der Verbindungen der allgemeinen Formel (1)
b) 1 - 40 Gew.-Teile mindestens eines nichtionischen, amphoteren, zwitterionischen, ionischen Tensids und
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

5. Wäßriges Geschirrspülmittel, enthaltend
a) 1 - 15 Gew.-Teile mindestens einer der Verbindungen der allgemeinen Formel (1) und
b) 1 - 40 Gew.-Teile mindestens eines Tensids aus der Gruppe der nichtionischen, amphoteren, zwitterionischen, ionischen Tenside und gegebenenfalls
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

6. Hautpflegemittel, enthaltend
a) 1 - 10 Gew.-Teile mindestens einer der Verbindungen der allgemeinen Formel (1) und
b) 1 - 10 Gew.-Teile mindestens eines Tensids aus der Gruppe der nichtionischen, amphoteren, zwitterionischen, ionischen Tenside und gegebenenfalls
c) 2 - 10 Gew.-Teile pflanzliche oder mineralische Oele, Esteroele
d) 1 - 5 Gew.-Teile Konsistenzgeber
e) 0,5 - 5,0 Gew.-Teile Duftstoffe, Farbstoffe, Konservierungsmittel
f) ad 100 Wasser.

7. Hautreinigungsmittel, enthaltend
a) 1 - 10 Gew.-Teile mindestens einer der Verbindungen der allgemeinen Formel (1) und
b) 0,1 - 20 Gew.-Teile mindestens eines Tensids aus der Gruppe der nichtionischen, amphoteren, zwitterionischen, ionischen Tenside und gegebenenfalls
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.
